# EUROPEAN PATENT SPECIFICATION

(11) **EP 3 355 788 B1**
(45) Date of publication and mention of the grant of the patent: **04.12.2019**
(21) Application number: 16798270.1
(22) Date of filing: 26.09.2016
(51) Int. Cl.: A61B 5/15, A61B 5/157, A61M 1/02, A61J 1/12, G01N 33/80

(54) **BIOMEDICAL CONTAINER TO COLLECT AND TEST BLOOD OR A BLOOD COMPONENT**
BIOMEDIZINISCHER BEHÄLTER ZUM SAMMELN UND TESTEN VON BLUT ODER EINER BLUTKOMPONENTE
RÉCIPIENT BIOMÉDICAL POUR COLLECTER ET ANALYSER DU SANG OU UN CONSTITUANT SANGUIN

(30) Priority: 30.09.2015 IT UB20154038
(43) Date of publication of application: 08.08.2018
(73) Proprietor: Iannini, Rocco, 12055 Diano d'Alba (IT); Pace, Cinzia, 16158 Genova (IT)
(72) Inventor: IANNINI, Rocco, 12055 Diano d'Alba (IT)
(74) Representative: Bergadano, Mirko
(86) International application number: PCT/IB2016/055749
(87) International publication number: WO 2017/055991

(56) References cited:
- EP-A2- 0 142 080
- WO-A1-2012/091655
- WO-A1-2014/010604
- DE-U1-202015 101 866
- GB-A- 989 885
- IT-B- 1 133 914

## Description

### Technical Field of the Invention

The present invention relates in general to the field of biomedical products for sampling and treating blood for transfusion purposes, and in particular to a biomedical container to collect and test blood or a blood component.

For the sake of descriptive convenience, the invention will be described with reference to a biomedical container in the form of a blood-bag (also known as blood sack or blood pouch), to which the following description will make reference without thereby losing in generality.

In fact, the present invention can equally find application also to biomedical containers different from a blood-bag under different aspects, particularly in terms of usage (disposable or multi-use), shape (bag or container of different shapes), material (relatively soft, semi-rigid or rigid), and blood product contained (blood or blood component).

Moreover, for the sake of conciseness, the following description will use the term blood-bag to refer both to a bag to collect blood and to a bag to collect a blood component, or a product derived from blood through simple fractionation or apheresis.

### State of the Art

The studies on adverse events related to blood transfusions have always shown that majority of transfusion accidents are correlated to blood group incompatibilities.

At present, blood group of red cells, intended for transfusion use, is determined from blood contained in a test tube and drawn from a donor at the time of donation and, later on, from blood in a blood-bag connecting tube after this has been physically separated from a blood-bag, after the blood bag has been welded and the connection tube has been cut. This procedure may cause an accidental exchange of test tubes or of connection tubes, which can result in a mistaken blood group typing of blood in the blood-bag.

Another issue relating to adverse events in transfusions is the potential bacterial contamination, particularly for blood components consisting of platelets. This unfavourable event is unlikely to be prevented, as is demonstrated by studies over the years that the risk of transmissible infections through blood have shown a significant reduction, but that the risk of transmission of diseases from bacterial contamination of the containers or the blood component has remained constant.

### Object and Summary of the Invention

The object of the present invention is to provide a blood-bag that allows the above-mentioned drawbacks to be overcome, so as to improve transfusion safety.

According to the present invention, a biomedical container is provided, as claimed in the appended claims.

### Brief Description of Drawings

Figure 1 depicts a blood-bag according to a first embodiment of the invention.
Figure 2 depicts a blood-bag according to a different embodiment of the invention.

### Detailed Description of the Preferred Embodiments of the Invention

The present invention will now be described in detail with reference to the attached figures to enable a skilled person to make it or use it. Various modifications of the described embodiments will be immediately apparent to those skilled in the art and the generic principles described may be applied to other embodiments and applications, without thereby deviating from the scope of protection of the invention, as defined in the appended claims. Therefore, the present invention should not be considered limited to the embodiment described and illustrated, but should be granted the widest scope in compliance with the principles and the features described and claimed herewith.

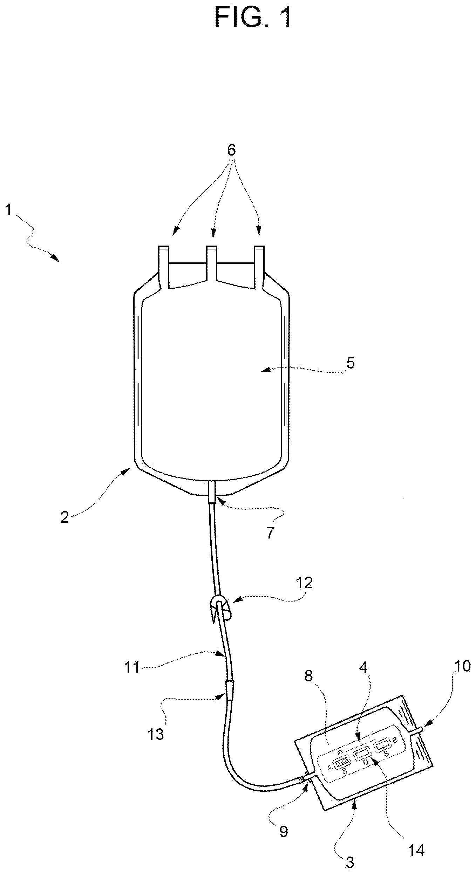

Figure 1 depicts a blood-bag, designated as a whole by reference numeral 1, according to a first embodiment of the present invention.

The blood-bag 1 essentially comprises:
- a collection blood-bag 2 to collect blood or a blood component supplied from the outside,
- one or more test blood-bags 3, only one of which is depicted in Figure 1 and each of which is connected to the collecting blood-bag 2 both mechanically, so as to be uniquely associated with the collecting blood-bag 2, and fluidically, in order to receive a sample of the blood or blood component collected in the collecting blood-bag 2, and
- a test kit 4 for each test blood-bag 3 and housed in the respective test blood-bag 3 to test the blood or blood component sample contained therein.

The collection blood-bag 2 is universally known and for this reason it will be described as far as the features necessary for the understanding of the present invention. In particular, here it is merely described that the collection blood-bag 2 is preferably made of a transparent material, in compliance with current laws and regulations, it defines an internal collection chamber 5 for collecting the blood or blood component, and comprises one or more fluidic connection ports 6 in its upper part - in the example depicted in Figure 1 in a number of three - and one or more fluidic connection ports 7 in its lower part - in the example depicted in Figure 1 in a number of one -- all of which are fluidically connected to the blood collection chamber 5 and can be used both as inlets and outlets.

During use, for example during blood sampling from a patient, one of the fluidic connection ports 6 of the collection blood-bag 2 is fluidically connected to one end of a blood sample/supply line, the opposite end of which is provided with a needle to be inserted in the patient's vein. Instead, the other fluidic connection ports 6 of the collection blood-bag 2 are typically designed to allow the collection blood-bag 2 to be fluidically connected to other blood-bags intended to collect different blood components derived from the blood collected in the collection blood-bag 2.

Each test blood-bag 3 is preferably made of a transparent material in compliance with current laws and regulations, defines an internal test chamber 8 for the blood or blood component and where a respective test kit 4 is arranged, and comprises one or more fluidic connection ports 9 in its upper part - in the example depicted in Figure 1. in a number of one - and one or more fluidic connection ports 10 in its lower part - in the example depicted in Figure 1 in a number of one - all of which are fluidically connected to the test chamber 8 and can be used both as inlets and outlets.

The collection chamber 5 and the test chamber 8 are fluidically connected in series, namely in such a way that the blood or the blood component flows first into the collection blood-bag 2 and then a sample thereof can be caused to flow from the collection blood-bag 2 to the test blood-bag 3. In particular, the test blood-bag 3 is fluidically connected to the collection blood-bag 2 via a fluidic connection line 11 in the form of a flexible tube, the ends of which are fluid-tightly connected to one of the fluidic connection ports 7 of the collection blood-bag 2 and, respectively, to one of the fluidic connection ports 9 of the test blood-bags 3.

In addition to fluidically connecting the test blood-bag 3 to the collection blood-bag 2, the fluidic connection line 11 also robustly and stably mechanically connect the test blood-bag 3 and the collection blood-bag 2 to prevent a possible unwanted physical separation thereof during normal use of the blood-bag 1. This mechanical connection results in a univocal association between the test blood-bag 3 and the collection blood-bag 2, thus making absolutely certain that the blood or blood component contained in the test chamber 8 and on which the test is performed is exactly the same as the one contained in the collection chamber 5.

The mechanical/fluidic connection of the test blood-bag 3 to the collection blood-bag 2 can alternatively be formed directly in the factory, whereby providing a blood-bag 1 right from the beginning, or otherwise *in situ,* when the need to form a blood-bag 1 arises. In the latter case, the collection blood-bag 2, possibly already provided with a blood sample/supply line connected to one of the fluidic connection ports 6, the test blood-bag 3 and the fluidic connection line 11 can be procured separately, or otherwise the test blood-bag 3 can be procured already fitted with the fluidic connection line 11, with the free end of which provided with a suitable coupling, for example a snap coupling, a press-fit coupling, or another equivalent coupling, which allows the fluidic connection line 11 to be mechanically and fluid-tightly fluidically connected to the collection blood-bag 2.

Conveniently, the mechanical/fluidic connection of the collection blood-bag 2 and the test blood-bag 3 to the fluidic connection line 11 is a damage-less unreleasable connection, namely such that the test blood-bag 3 cannot be subsequently separated from the collection blood-bag 2 without damaging the test blood-bag 3 and/or the collection blood-bag 2 and/or the fluidic connection line 11, whereby the mechanical/fluidic connection cannot later be restored. Such a mechanical/fluidic connection may conveniently be achieved by welding the ends of the fluidic connection line 11 to one of the fluidic connection ports 7 of the collection blood-bag 2 and to one of the fluidic connection ports 9 of the test blood-bag 3.

Alternatively, the mechanical/fluidic connection of the collection blood-bag 2 and of the test blood-bag 3 to the fluidic connection line 11 could also be a damage-less releasable connection, namely such that the test blood-bag 3 can be separated from the collection blood-bag 2 without damaging to the test blood-bag 3 and/or the collection blood-bag 2 and/or the fluidic connection line 11, whereby the mechanical/fluidic connection can later be restored. Such a mechanical/fluidic connection could for example be achieved via fluid-tight snap couplings, fluid-tight press-fit couplings, or other equivalent couplings.

Both embodiments could then conveniently be provided with an anti-tamper device to allow an operator to check a possible separation of the test blood-bag 3 from the collection blood-bag 2 and a subsequent restoration of the connection. Along the fluidic connection tube (11) there may also be provided:
- one or more clamps 12, only one of which is depicted in Figure 1 and each of which is manually operable by an operator to shut the fluidic connection line 11 and interrupt the fluidic connection between the collection blood-bag 2 and the test blood-bag 3 after a blood or blood component sample has flowed from the collection blood-bag 2 to the test blood-bag 3, whereby preventing the blood or blood component sample from flowing back toward the collection blood-bag 2, or, on the contrary, to open the fluidic connection line 11 and allow a blood or blood component sample to flow from the collection blood-bag 2 to the test blood-bag 3; and, possibly,
- a hand-breakable circuit opener 13, which is normally closed, is arranged downstream from the clamp 12 and is intended to be broken manually by an operator only after the fluidic connection line 11 has collected a quantity of blood or blood component sufficient for it to be tested in the test blood-bag 3, and the upstream clamp 12 has been closed and the fluidic connection line 11 has been possibly welded by means of a blood-bag welder to prevent a blood back flow into the collection blood-bag 2.

Alternatively, the function of the circuit opener 13 can be carried out by a second clamp 12, or alternatively the clamp 12 and the circuit opener 13 may potentially be replaced with any other system designed for that purpose.

The test kit 4 can be of any type, depending on the test to be performed on the blood or blood component contained in the test blood-bag 3, and it may have any shape and structure.

Conveniently, the test kit 4 may be a kit to perform an immuno-haematological test, and/or an analyte test and/or a bacterial test. The latter could for example be of the type based on a bacterial culture verification, such as for blood platelets.

Whatever the type, structure and conformation, the test kit 4 is of the type provided with display means 14 intended to allow an operator to appreciate the test result.

By way of a non-limiting example, Figure 1 depicts a test kit 4 in the form of an ABO Blood Grouping Test Card or Cassette, which, as is known, contains the antisera necessary to determine the blood type in liquid adhesed or adsorbed form on suitable supports (cards, mini gel columns, etc.), and is provided with passive display means 14 to display the test result and in the form of areas suitably treated to highlight different reactions for different test results, which display means 14 are visible from the outside of the test blood-bag 3 thanks to the fact that this is made of a transparent material.

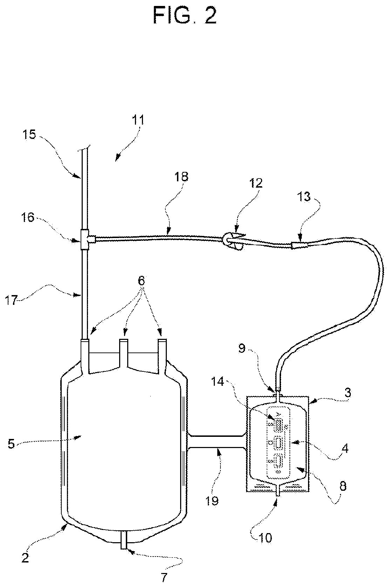

Figure 2 depicts a different embodiment of the present invention, which will be described as far as the differences compared to the embodiment depicted in Figure 1 and therefore using the same reference numerals to identify the same components.

In the embodiment depicted in Figure 2, the fluidic and mechanical connections of the test blood-bag 3 to the collection blood-bag 2 have the same features as those in the embodiment depicted in Figure 1, and differ from these in that the fluidic connection is such that it fluidically connects in parallel, rather than in series, the collection blood-bag 2 to the test blood-bag 3, and in that the mechanical connection, which results in the univocal association of the test blood-bag 3 to the collection blood-bag 2, can alternatively be achieved by using the same components that form the fluidic connection, as in the embodiment depicted in Figure 1, or otherwise through components that are distinct from those that form the fluidic connection.

In particular, the fluidic connection line 11 that fluidically connects the test blood-bag 3 to the collection blood-bag 2 is in the form of a flexible branched piping arrangement comprising a flexible input branch 15 intended to be flown by the blood or blood component, and from which two output branches 17, 18 branch off through a suitable fluidic branch 16, for example in the form of a fluidic T or Y junction, and which are fluid-tightly connected to one of the fluidic connection ports 6 of the collection blood-bag 2 and, respectively, to one of the fluidic connection ports 9 of the test blood-bag 3.

In this way, during use, the blood or the blood component flows partly into the collection blood-bag 2 and partly into the test blood-bag 3, whereby the blood or blood component being tested in the test blood-bag 3 is the same as the one collected in the collection blood-bag 2, without however having flowed through latter.

The mechanical connection of the test blood-bag 3 to the collection blood-bag 2 can instead be alternatively formed by exploiting the same flexible piping arrangement, or by using distinct connecting components. In particular, in the first case, the mechanical connection can be conveniently formed using the two output branches 17, 18, after prior occlusion of the inlet branch 15, by conveniently welding the inlet branch 15 with a blood-bag welder. In the second case, instead, the mechanical connection can for example be formed by welding a strip 19 to the collection blood-bag 2 and the test blood-bag 3, conveniently made of the same material as that of the collection blood-bag 2 and the test blood-bag 3, or even by directly connecting, once again conveniently by welding, sections of the perimeter edges of the collection blood-bag 2 and of the test blood-bag 3.

Even in this embodiment, the mechanical/fluidic connection of the test blood-bag 3 to the collection blood-bag 2 can alternatively be formed directly in factory, whereby providing a blood-bag 1 right from the beginning, or otherwise *in situ,* when the need to form a blood-bag 1 arises. In the latter case, the collection blood-bag 2, possibly already provided with a blood sample/supply line connected to one of the fluidic connection ports 6, the test blood-bag 3, the fluidic connection line 11 and, if provided, the connecting strip 19, can be procured separately, or the test blood-bag 3 can be procured already fitted with the fluidic connection line 11, with the free end provided with a suitable coupling, for example a snap coupling, a press-fit coupling or other equivalent couplings, which allows the fluidic connection line 11 to be fluid-tightly fluidically connected to the collection blood-bag 2, or even the test blood-bag 3 can be procured provided with only part of the fluidic connection line 11, for example with only the output branch 18, to be successively fluidically connected to the fluidic branch 16, which may be part of another separate fluidic line already connected or to be connected to the collection blood-bag 2. When the fluidic connection line 11 is procured separately, it could be manufactured directly in factory or *in situ,* when the need to form a fluidic connection line 11 arises. In the latter case, the components of the fluidic connection line 11 can be procured separately or already suitably connected to form separate parts of the fluidic connection line 11.

From the above description, the advantages that the blood-bag according to the present invention allows to achieve may be appreciated.

In particular, the possibility of determining the blood group, the absence or presence of any bacterial growth or any other physiochemical parameter of blood or of a blood component in a test blood-bag univocally associated and undetachably mechanically connected to the blood-collecting bag and that, for this reason, contains the same blood or blood component collected in the collection blood-bag, allows an operator to determine with absolute certainty, and at any time, the belonging of the blood or blood component contained in the collection blood-bag to a particular blood group, the absence of bacterial growth or the outcome of the test performed.

This consequently results in:
- an increased safety in transfusion, by reducing for example the risk of the incorrect determination of the blood group collected in the collection blood-bag and by determining any bacterial contamination of the blood or blood component collected in the collection blood-bag, and
- a reduced contamination risk for operators, who no longer have to intervene mechanically on the connecting tubes to sample the blood or blood component.

What is previously described and illustrated can be subject to modifications and variations without thereby departing from the protective scope of the present invention, as defined in the appended claims.

For example, the test blood-bag may be made with a different material or may have a different shape from those described and illustrated, thus adapting to any test kit to be arranged therein.

Moreover, instead of being made of two separate blood-bags, the collection and test chambers can both be formed within one and the same blood-bag, which must therefore be suitably designed for this purpose and so as to achieve the anti-reflux function carried out by the clamps and circuit openers. In this embodiment, formation of both collection and test chambers in the same blood-bag automatically guarantees the univocal association of the test chamber to the collection chamber and, resultingly, the absolute certainty that the blood or blood component collected in the test chamber, and which is subjected to test, is exactly the same as the one collected in the collection chamber.

In addition, either one or both of collection and test chambers may be made formed in a single blood-bag or in different blood-bags made of a non-transparent material. When the test chamber is formed in a blood-bag made of a non-transparent material, the result of the test on the blood or blood component collected therein can be made visible from the outside by forming a special window in a position facing the test result display means.

Finally, the test result display means may be different from those described and illustrated. In particular, should the type of biomedical container used so allow, the display means may also be of an active type, such as, for example, comprising an electronic display, or based on RFID technology.

## Claims

1. A biomedical container (1) to collect and test blood or a blood component, comprising:
- a collection chamber (5) for blood or a blood component;
- a test chamber (8) for blood or a blood component; and
- a test kit (4) in the test chamber (8) to test the blood or the blood component contained therein;
wherein the collection chamber (5) and the test chamber (8) are intended to be connected so as to be unequivocally mutually associated and to be supplied with the same blood or blood component.

2. A biomedical container (1) according to claim 1, wherein the collection chamber (5) and the test chamber (8) are intended to be fluidically connected in series with each other.

3. A biomedical container (1) according to claim 2, wherein the collection chamber (5) and the test chamber (8) are defined by a collection receptacle (2) and, respectively, by a test receptacle (3) which are distinct and connectable to each other by means of a fluidic connection line (11) defining, in addition to a fluidic connection between the collection chamber (5) and the test chamber (8), also a mechanical connection of the test receptacle (3) to the collection receptacle (2) and, resultingly, their unequivocal mutual association.

4. A biomedical container (1) according to claim 1, wherein the collection chamber (5) and the test chamber (8) are intended to be fluidically connected in parallel to each other.

5. A biomedical container (1) according to claim 4, wherein the collection chamber (5) and the test chamber (8) are defined by a collection receptacle (2) and, respectively, by a test receptacle (3) which are separate, mechanically connected to each other to define their unequivocal mutual association, and fluidically connectable to each other via a fluidic connection line (11) in the form of a branched piping arrangement comprising an inlet branch (15) for blood or a blood component and from which two outlet branches (17, 18), which are connectable to the collection receptacle (2) and, respectively, to the test receptacle (3) branch off through a fluid branch (16).

6. A biomedical container according to claim 3 or 5, wherein the collection chamber (5) and the test chamber (8) are each in the form of a blood bag.

7. A biomedical container according to any one of the preceding claims, wherein the test kit (4) is an immune-haematological and/or haemochemical and/or bacterial blood test kit.

8. A biomedical test receptacle (3) containing a test kit (4) to test blood or a blood component and intended to be connected to a biomedical collection receptacle (2) to form a biomedical container (1) according to any one of the preceding claims.

9. A biomedical test receptacle (3) according to claim 8, also comprising a fluidic connection line (11) to connect the biomedical test receptacle (3) to the biomedical collection receptacle (2).

10. A method of collecting and testing blood or a blood component, comprising:
- providing a collection receptacle (2) defining a collection chamber (5) for blood or a blood component;
- providing a test receptacle (3) defining a test chamber (8) containing a test kit (4) to test blood or a blood component; and
- connecting the collection receptacle (2) and the test receptacle (3) so as to be unequivocally mutually associated and to be supplied with the same blood or blood component.

## Patentansprüche

1. Biomedizinischer Behälter (1) zum Sammeln und Testen von Blut oder einer Blutkomponente, der aufweist:
eine Sammelkammer (5) für Blut oder eine Blutkomponente;
eine Testkammer (8) für Blut oder eine Blutkomponente; und
ein Testkit (4) in der Testkammer (8), um das darin enthaltene Blut oder die Blutkomponente zu testen;
wobei die Sammelkammer (5) und die Testkammer (8) dazu bestimmt sind, so verbunden zu sein, dass sie einander eindeutig zugeordnet sind und mit dem gleichen Blut oder der gleichen Blutkomponente versorgt werden.

2. Biomedizinischer Behälter (1) nach Anspruch 1, wobei die Sammelkammer (5) und die Testkammer (8) dazu bestimmt sind, in Reihe miteinander fluidverbunden zu sein.

3. Biomedizinischer Behälter (1) nach Anspruch 2, wobei die Sammelkammer (5) und die Testkammer (8) durch ein Sammelbehältnis (2) bzw. durch ein Testbehältnis (3) gebildet sind, die sich voneinander unterscheiden und mittels einer Fluidverbindungsleitung (11) miteinander verbindbar sind, die zusätzlich zu einer Fluidverbindung zwischen der Sammelkammer (5) und der Testkammer (8) auch eine mechanische Verbindung des Testbehältnisses (3) mit dem Sammelbehältnis (2) und als Ergebnis ihre eindeutige Zuordnung zueinander bildet.

4. Biomedizinischer Behälter (1) nach Anspruch 1, wobei die Sammelkammer (5) und die Testkammer (8) dazu bestimmt sind, parallel zueinander fluidverbunden zu sein.

5. Biomedizinischer Behälter (1) nach Anspruch 4, wobei die Sammelkammer (5) und die Testkammer (8) durch ein Sammelbehältnis (2) bzw. durch ein Testbehältnis (3) gebildet sind, die getrennt, miteinander mechanisch verbunden, um ihre eindeutige Zuordnung zueinander zu bilden, und über eine Fluidverbindungsleitung (11) in Form einer verzweigten Rohranordnung miteinander fluidverbindbar sind, die einen Einlasszweig (15) für Blut oder eine Blutkomponente aufweist und von der zwei Auslasszweige (17, 18), die mit dem Sammelbehältnis (2) bzw. mit dem Testbehältnis (3) verbindbar sind, über einen Fluidzweig (16) abzweigen.

6. Biomedizinischer Behälter nach Anspruch 3 oder 5, wobei die Sammelkammer (5) und die Testkammer (8) jeweils die Form eines Blutbeutels haben.

7. Biomedizinischer Behälter nach einem der vorstehenden Ansprüche, wobei das Testkit (4) ein immunhämatologisches und/oder hämochemisches und/oder bakterielles Blut-Testkit ist.

8. Biomedizinisches Testbehältnis (3), das ein Testkit (4) zum Testen von Blut oder einer Blutkomponente enthält und dazu bestimmt ist, mit einem biomedizinischen Sammelbehältnis (2) verbunden zu sein, um einen biomedizinischen Behälter (1) nach einem der vorstehenden Ansprüche zu bilden.

9. Biomedizinisches Testbehältnis (3) nach Anspruch 8, das ferner eine Fluidverbindungsleitung (11) aufweist, um das biomedizinische Testbehältnis (3) mit dem biomedizinischen Sammelbehältnis (2) zu verbinden.

10. Verfahren zum Sammeln und Testen von Blut oder einer Blutkomponente, das aufweist:
Bereitstellen eines Sammelbehältnisses (2), das eine Sammelkammer (5) für Blut oder eine Blutkomponente bildet;
Bereitstellen eines Testbehältnisses (3), das eine Testkammer (8) bildet, die ein Testkit (4) enthält, um Blut oder eine Blutkomponente zu testen; und
Verbinden des Sammelbehältnisses (2) und des Testbehältnisses (3), damit sie einander eindeutig zugeordnet sind und mit dem gleichen Blut oder der gleichen Blutkomponente versorgt werden.

## Revendications

1. Un conteneur biomédical (1) pour collecter et tester du sang ou un composant sanguin, comprenant:
- une chambre de collecte (5) pour le sang ou un composant sanguin;
- une chambre de test (8) pour le sang ou un composant sanguin; et
- un kit de test (4) dans la chambre de test (8) pour tester le sang ou le composant sanguin contenu dans celle-ci;
dans lequel la chambre de collecte (5) et la chambre de test (8) sont destinées à être connectées de manière à être associées sans équivoque mutuellement et à être alimentées avec le même sang ou le même composant sanguin.

2. Un conteneur biomédical selon la revendication 1, dans lequel la chambre de collecte (5) et la chambre de test (8) sont destinées à être connectées de manière fluidique en série l'une avec l'autre.

3. Un conteneur biomédical selon la revendication 2, dans lequel la chambre de collecte (5) et la chambre de test (8) sont définies par un réceptacle de collecte (2) et, respectivement, par un réceptacle de test (3) qui sont distincts et peuvent être connectés l'un à l'autre au moyen d'une conduite de connexion fluidique (11) définissant, en plus d'une connexion fluidique entre la chambre de collecte (5) et la chambre de test (8), également une connexion mécanique du récipient de test (3) au réceptacle de collecte (2) et, par conséquent, leur association mutuelle sans équivoque.

4. Un conteneur biomédical (1) selon la revendication 1, dans lequel la chambre de collecte (5) et la chambre de test (8) sont destinées à être connectées de manière fluidique en parallèle l'une à l'autre.

5. Un conteneur biomédical (1) selon la revendication 4, dans lequel la chambre de collecte (5) et la chambre de test (8) sont définies par un réceptacle de collecte (2) et, respectivement, par un réceptacle de test (3) qui sont séparés, mécaniquement connectées entre elles pour définir leur association mutuelle non équivoque, et connectables de manière fluidique l'une à l'autre via une conduite de connexion fluidique (11) se présentant sous la forme d'un ensemble de conduites ramifiées comprenant une branche d'entrée (15) pour le sang ou un composant sanguin et à partir de laquelle deux branches de sortie (17, 18), qui sont connectables au réceptacle de collecte (2) et, respectivement, au réceptacle de test (3), sont dérivées par une branche fluidique (16).

6. Un conteneur biomédical selon la revendication 3 ou 5, dans lequel la chambre de collecte (5) et la chambre de test (8) sont chacune sous la forme d'une poche de sang.

7. Un conteneur biomédical selon l'une quelconque des revendications précédentes, dans lequel le kit de test (4) est un kit de test sanguin immuno-hématologique et / ou hémochimique et / ou bactérien.

8. Un réceptacle de test biomédical (3) contenant un kit de test (4) pour tester le sang ou un composant sanguin et destiné à être connecté à un réceptacle de collecte biomédical (2) pour former un conteneur biomédical (1) selon l'une quelconque des revendications précédentes.

9. Un réceptacle de test biomédical (3) selon la revendication 8, comprenant également une ligne de connexion fluidique (11) pour connecter le réceptacle de test biomédical (3) au réceptacle de collecte biomédical (2).

10. Procédé de collecte et de test de sang ou d'un composant sanguin, comprenant:
- la fourniture d'un réceptacle de collecte (2) définissant une chambre de collecte (5) pour le sang ou un composant sanguin;
- fournir un réceptacle de test (3) définissant une chambre de test (8) contenant un kit de test (4) pour tester le sang ou un composant sanguin; et
- connecter le réceptacle de collecte (2) et le réceptacle de test (3) de manière à être mutuellement associés sans équivoque et à recevoir le même sang ou le même composant sanguin.
